# EUROPEAN PATENT SPECIFICATION

(11) **EP 0 626 837 B1**
(45) Date of publication and mention of the grant of the patent: **09.04.1997**
(21) Application number: 93904457.4
(22) Date of filing: 16.02.1993
(51) Int. Cl.: A61F 13/15

(54) **AN ABSORBENT DISPOSABLE ARTICLE CONSTRUCTED FROM AT LEAST TWO FLAT FLEXIBLE BODIES CONTAINING ABSORBENT MATERIAL**
ABSORBIERENDER EINWEGARTIKEL HERGESTELLT AUS ZUMINDEST ZWEI FLEXIBLEN FLÄCHEN, WELCHE EIN ABSORBIERENDES MATERIAL ENTHALTEN
ARTICLE ABSORBANT JETABLE CONSTITUE D'AU MOINS DEUX ELEMENTS SOUPLES PLATS CONTENANT UN MATERIAU ABSORBANT

(30) Priority: 18.02.1992 SE 9200482
(43) Date of publication of application: 07.12.1994
(73) Proprietor: SCA Mölnlycke AB, 405 03 Göteborg (SE)
(72) Inventor: WIDLUND, Urban, S-435 43 Mölnlycke (SE)
(74) Representative: Kierkegaard, Lars-Olov
(86) International application number: SE9300120
(87) International publication number: WO9315700

(56) References cited:
- US-A- 3 884 234

## Description

The present invention relates to an absorbent disposable article, such as a sanitary napkin, a panty protector, a diaper or an incontinence guard.

Such articles are normally flat, i.e. have a two-dimensional shape, and are manufactured from flexible material. Although the flat shape of such articles is convenient from the technical aspect of manufacture, a flat article cannot readily be caused to conform to the shape of the wearer's anatomy. Because a poorly-fitting article will, in many cases, give rise to leakage and subsequent discolouring of the wearer's underclothes, various attempts have been made to produce articles of the aforesaid kind which will adapt better to the shape of the wearer's anatomy. For instance, the flat articles have been provided in manufacture with pre-stretched elastic which, when contracting, forces the two-dimensional article to take a three-dimensional shape. One advantage with this solution is that the articles can be packed in a flat state, although the provision of elastication results in more complicated and expensive manufacture.

Another solution for providing a three-dimensional shape in sanitary napkins or like articles is to impart the desired shape to the sanitary napkin during the manufacturing process. An example of this solution is given in EP-A2-0302523. One drawback with this solution is that articles which are three-dimensional from the beginning are relatively expensive to manufacture and, consequently, the purchase price of such articles is relatively high.

US-A-3,884,234 discloses a diaper with a fold line and a pocket formed thereby in the rear portion.

The object of the present invention is to provide an absorbent disposable article which has a flat packaging state and a three-dimensional state of use and which in its state of use will conform effectively with the shape of the wearer's body.

This object is achieved in accordance with the invention with an absorbent disposable article of the aforesaid kind which is characterized in that the article comprises at least two generally flat, flexible bodies which include absorbent material and which in a packaging state can lie against one another with first flat sides which are intended to face towards the wearer when the article is in use; in that two neighbouring bodies of the article are mutually joined along a first common edge which forms a folding line around which the bodies can be folded out from their packaging state, and which includes an arcuate section which in the packaging state of the article is arched towards a second, opposing edge of the bodies. An article of this kind can be manufactured generally in the same manner as conventional flat articles and may also be packed flat, with the inner surfaces of the bodies of the article in mutual abutment with one another. When the absorbent bodies of the article are folded out from their packaging state so as to mutually separate the abutting sides of the bodies, there is formed a three-dimensional article which has a part which is outwardly concave, i.e. inwardly convex or curved toward the wearer, within the region of the arcuate edge-section. This outwardly concave part can readily be fashioned to a full or a partial outwardly convex configuration with a simple hand movement, whereupon the article within this manually curved area obtains a cross-sectional shape in the form of an inverted V. A sanitary napkin having a part which is shaped in this way will fit snugly around the wearer's body, within the region where the buttocks meet.

The invention will now be described in more detail with reference to a preferred embodiment thereof and also with reference to the accompanying drawings, in which
Figure 1 is a side view of an inventive absorbent article;
Figure 2 is a perspective view obliquely from above which illustrates the article in Figure 1 when un-folded;
Figure 3 is a view similar to the view of Figure 2 and shows the article when ready for use;
Figure 4 is a view similar to the view of Figure 2 and illustrates another conceivable shaped form of the article; and
Figure 5 illustrates in perspective a packaging stack of articles shown in Figure 1.

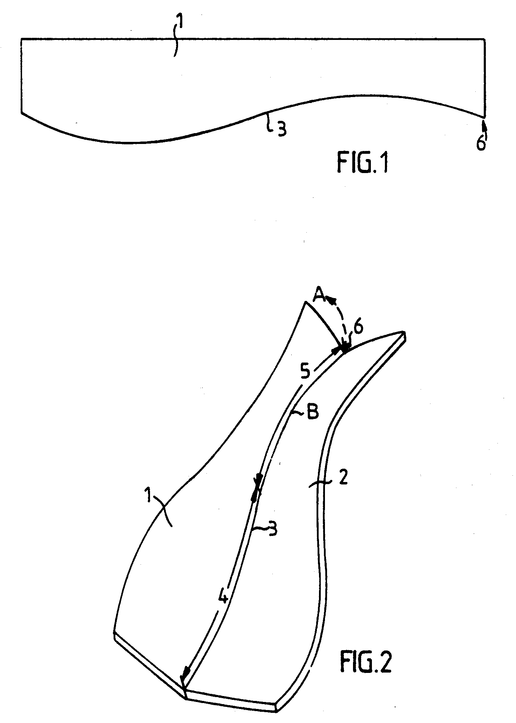

Figures 1-4 illustrate schematically a sanitary napkin configured in accordance with a preferred embodiment of the invention. The illustrated napkin is comprised of two elongated, mirror-symmetrical absorbent bodies 1, 2, of which only the body 1 can be seen in Figure 1. The bodies 1, 2 are mutually joined along an S-shaped edge 3 which extends in the longitudinal direction of the napkin.

The bodies 1, 2 are preferably constructed in a conventional manner, i.e. with an absorbent pad enclosed between an inner liquid-permeable casing sheet which in use lies nearest the wearer's skin and an outer liquid-impermeable casing sheet, although it can also be made of homogenous bonded material. As opposed to the thin elastic casing materials normally used to produce flat absorbent pads for diapers, sanitary napkins or incontinence guards, the outer casing sheet included in the bodies 1, 2 may consist of a much stiffer, cheaper material, which does not need to be elastic. The stiffness of this material, however, will preferably not prevent the material from being wound onto and unwound from a material roll so as to enable a web of outer sheet material used in accordance with the invention to be used in the manner conventional in the manufacture of flat absorbent disposable articles.

Hydrophobic non-woven material or plastic film, for instance polyethylene film, are suitable outer casing materials.

The inner casing sheet may consist of a non-woven material or like material, for instance thermobonded spunbonded material or carded non-woven.

The absorbent pad enclosed between the casing sheets may consist of a cellulose fibre body which may or may not contain an addition of so-called superabsorbents and/or thermofibres. However, because the bodies 1, 2 shall be thin, it is preferred to mix superabsorbent particles with the cellulose fibres so as to increase the amount of liquid that can be absorbed, i.e. the maximum amount of liquid that can be absorbed in the absorbent pad. In such cases, an insulating layer can be placed conveniently between the inner casing sheet and the fibre body, partly to reduce the risk of rewetting and partly to increase the softness of that part of the napkin which lies closest to the wearer's body.

Naturally, the absorbent pad can be constructed in other ways without departing from the scope of the invention. For instance, the absorbent material in the absorbent pad may consist solely of superabsorbents disposed in or between appropriate diffusion layers.

Figure 2 shows the napkin of Figure 1 unfolded. As will be seen from Figure 2, when the napkin is unfolded a cupped part is formed in the napkin within the region of the outwardly (downwardly in the Figures) convex section 4 of the S-shaped edge 3. It will also be seen that the article has a V-shaped cross-section within the region of the outwardly concave section 5 of the S-shaped edge.

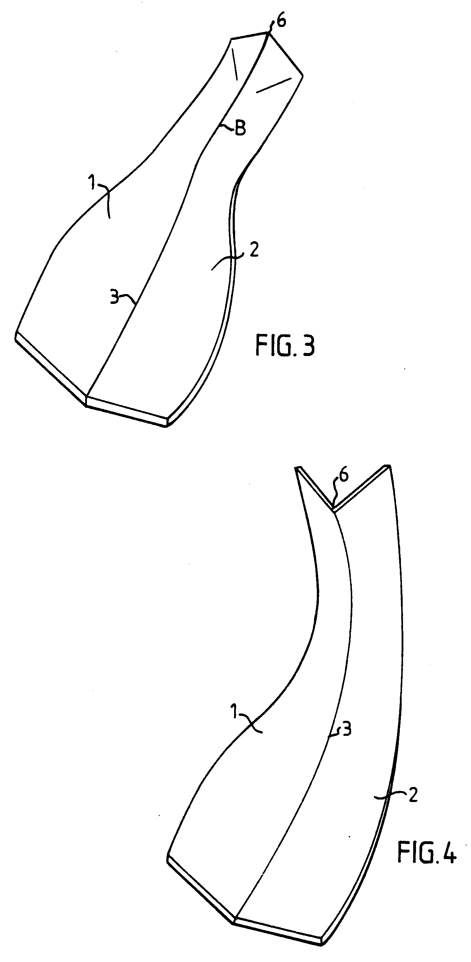

Figure 3 illustrates the napkin in its ready-for-use state, i.e. its state prior to being placed on the wearer's body. The configuration of the napkin in its state of use differs from the configuration achieved solely by folding out the napkin in that the downwardly extending end-part (adjacent edge 6) of the outwardly concave section 5 of the edge 3, as seen in Figures 1 and 2, is curved upwardly in the Figure 3 configuration, and in that the napkin in cross-section has the shape of an inverted V within this part of the napkin. Furthermore, within the immediately following part of the section 5, the bodies 1, 2 are almost fully unfolded, i.e. the article is essentially flat within this part of the napkin.

This upward bending of the edge 3 in the aforesaid end-part of the section 5 can be achieved with a simple hand movement, namely by placing a thumb on the point B in Figure 2 and then pressing the end 6 of the edge 3 upwards with the index finger, as indicated by the broken arrow A. The end-part of the section 5 will then automatically be brought to the shape shown in Figure 3, because this shape constitutes one of the stable shapes permitted by the geometrical configuration of the napkin. Figure 4 illustrates another stable shape of the napkin, in which the whole of that section 5 of the edge 3 which was originally outwardly concave is curved upwards. It is also possible to give the napkin a stable shape when solely the forward part of the section 5 is curved upwards while the end-part of the section 5 has the shape shown in Figure 2. It will be seen that the upwardly curved end-part of the section 5 in Figure 3 can be given an appropriate size, by varying the curvature of the outwardly concave section 5 of the S-shaped edge. It will also be seen that by composing the outwardly concave section 5 of the edge 3 of portions of mutually different curvatures, optionally including straight parts, it is possible to provide an article which can be given a number of different stable shapes. The term "arcuate", as used in the following Claims, shall not therefore be given a limited interpretation and shall be considered to include a composite curve form of the outwardly concave section 5 of the edge 3.

It should be mentioned in this connection that the outwardly convex shape of the edge 3 within section 4 is not a necessary feature of the invention and that this edge section may conceivably have other shapes, such as a straight shape for instance. The illustrated shape is preferred, however, since the resultant cupped shape of the forward part of the napkin provides a particularly good body fit.

It will be seen from Figure 3 that the point B lies forwardly of the upwardly curved part of the edge section 5, which is appropriate. Seen generally, how-ever, this is not a prerequisite for automatically obtaining the napkin configuration shown in Figure 3 as the end 6 of the edge 3 is curved upwards. When the stiffness of the outer casing sheet of the napkin approaches the limit at which the article is shaped stable, however, the point B must lie forwardly of the upwardly curved part of the section 5 in Figure 3, in order to obtain the desired shape. Furthermore, it may be necessary to place the index finger on the underside of the napkin opposite the point B and opposite the thumb, and then move the index finger backwards and upwards. A similar hand movement is also necessary in obtaining the shape shown in Figure 4.

In the described embodiment of the invention, the requisite rigidity of the body is obtained by means of the outer casing sheet which supports the absorbent pad and the inner casing sheet and imparts shape stability to the composite body. It will be understood, however, that it is the total rigidity and flexibility of the composite bodies included in the article that are of significance. It is therefore possible within the scope of the present invention to utilize, for instance, an outer casing sheet of conventional kind which lacks essential stiffness and to provide the absorbent pad sandwiched between the casing layers with the requisite stiffness and flexibility, either by using suitable inserts or the like, or even by providing an absorbent pad in which has these properties are intrinsic. In the aforegoing, the invention has been described with reference to a sanitary napkin in which the upwardly curved end-part of the napkin with its inverted-V form enables the article to be fitted conformingly to the region between the buttocks of the wearer. It will be understood, however, that the inventive principle can also be applied to other absorbent disposable articles. For instance, the configuration illustrated in Figure 4 may be suitable for the crotch and rear part of a disposable diaper. It will be understood that when the invention is applied to diapers, which extend further up the back of the wearer than does a sanitary napkin, the rear part of the diaper will not be terminated with an outwardly concave part. In order to ensure that the V-shape illustrated in Figure 3 can be obtained in the case of diapers, the diaper should be provided with two sequential outwardly concave sections in the area intended so as to form two opposing V-shaped regions which together form a pyramid when the concave parts of the diaper are curved upwardly in a manner similar to that described with reference to Figures 1-3.

The invention thus provides an absorbent disposable article which can be manufactured and packaged in a flat state and used in a three-dimensional state while ensuring that the article will fit conformingly to the body of the wearer. When taken together, these properties of the inventive article enable the article to be manufactured at a reasonable cost and therewith retailed at a reasonable price.

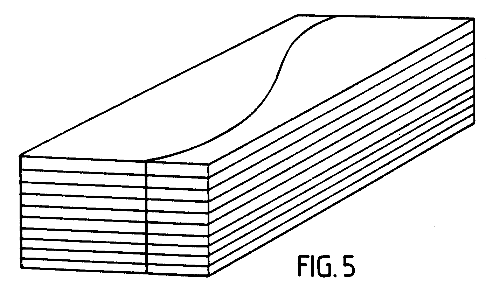

Articles having the configuration illustrated in Figure 1 and comprising of two mirror-symmetrical bodies having a common S-shaped edge can be packaged in a particularly space-saving fashion when the two arcuate sections forming the S-shape are mirror-symmetrical in relation to a line that passes through the ends of the S, by placing the articles side-by-side in the manner shown in Figure 5, i.e. with the convex section of one article being placed in the concave section of the other article. The stack of articles shown in Figure 5 is placed in a bag or some like packaging medium.

## Claims

1. An absorbent disposable article such as a sanitary napkin, a panty protector, a diaper or an incontinence guard, **characterized** in that the article comprises at least two generally flat, flexible bodies (1, 2) which include absorbent material and which in a packaging state can lie against one another with first flat sides which are intended to face towards the wearer when the article is in use; in that two neighbouring bodies (1, 2) of the article are mutually joined along a first common edge (3) which forms a folding line around which the bodies can be folded out from their packaging state and which includes an arcuate section (5) which, in the packaging state of the article, is arched towards a second opposing edge of the bodies (1, 2).

2. An article according to Claim 1, **characterized** in that it is composed of a pair of bodies (1, 2) which are mirror-symmetrical in the longitudinal direction of the article.

3. An article according to Claim 1 or 2, **characterized** in that the mutually joined edges (3) of the bodies (1, 2) are S-shaped.

4. An article according to any one of Claims 1-3, **characterized** in that the bodies (1, 2) are comprised of an outer, liquid-impermeable casing material, an inner, liquid-permeable casing material which is intended to lie nearest the wearer's body when the article is worn, and an absorbent pad enclosed between the casing sheets.

5. An article according to any one of the preceding Claims, **characterized** in that the bodies are two mirror-symmetrical bodies (1, 2), having a common S-shaped edge (3), and in that the two arcuate sections which form the S-shape are mirror-symmetrical in relation to a line passing through the ends of said sections.

## Patentansprüche

1. Absorbierender Einwegartikel, wie beispielsweise eine Damenbinde, eine Slipeinlage, eine Windel oder ein Inkontinenzschutz,
**dadurch gekennzeichnet, daß**
der Artikel zumindest zwei im wesentlichen flache, flexible Teile (1, 2) umfaßt, die absorbierendes Material beinhalten und die in einem Verpackungszustand mit ersten flachen Seitenflächen gegeneinander liegen können, die dazu bestimmt sind, zum Träger hin zu zeigen, wenn der Artikel in Benutzung ist, daß zwei nebeneinanderliegende Teile (1, 2) des Artikels entlang einer gemeinsamen Kante (3) miteinander verbunden sind, die eine Faltlinie bildet, um welche die Teile aus deren Verpackungszustand auseinandergefaltet werden können, und die einen bogenartigen Abschnitt (5) beinhaltet, der im Verpackungszustand des Artikels in Richtung einer zweiten gegenüberliegenden Kante der Teile (1, 2) gebogen ist.

2. Artikel nach Anspruch 1,
**dadurch gekennzeichnet, daß**
er aus einem Paar Teile (1, 2) gebildet ist, die in Längsrichtung des Artikels spiegelsymmetrisch ausgebildet sind.

3. Artikel nach Anspruch 1 oder 2,
**dadurch gekennzeichnet, daß**
die miteinander verbundenen Kanten (3) der Teile (1, 2) S-förmig sind.

4. Artikel nach einem der Ansprüche 1 - 3,
**dadurch gekennzeichnet, daß**
sich die Teile (1, 2) aus einem äußeren, flüssigkeitsundurchlässigen Hüllmaterial, einem inneren, flüssigkeitsdurchlässigen Hüllmaterial, das dazu bestimmt ist, nächst dem Körper des Trägers zu liegen, wenn der Artikel getragen wird, und einem zwischen den Hüllagen eingeschlossenen absorbierenden Kissen zusammensetzen.

5. Artikel nach einem der vorhergehenden Ansprüche,
**dadurch gekennzeichnet, daß**
die Teile zwei spiegelsymmetrische Teile (1, 2) sind, die eine gemeinsame S-förmige Kante (3) aufweisen, und daß die zwei bogenartigen Abschnitte, welche die S-Form bilden, bezüglich einer durch die Enden der Abschnitte gehende Linie spiegelsymmetrisch sind.

## Revendications

1. Article absorbant jetable comme une serviette hygiénique, un protège-slip, une couche-culotte ou une protection pour incontinents, caractérisé en ce que l'article comprend au moins deux corps souples (1, 2), généralement plats, qui contiennent un matériau absorbant et qui, dans une situation d'emballage, peuvent être placés l'un conte l'autre avec des premiers côtés plats qui sont destinés à se trouver face à l'utilisateur quand l'article est porté, en ce que deux corps voisins (1, 2) de l'article sont réunis l'un à l'autre le long d'un premier bord commun (3) qui forme une ligne de pliage autour de laquelle les corps peuvent être dépliés de leur situation d'emballage et qui comporte une partie courbe (5) qui, lorsque l'article est dans la situation d'emballage, est arquée en direction d'un second bord, opposé, des corps (1, 2).

2. Article selon la revendication 1, caractérisé en ce qu'il est composé d'une paire de corps (1, 2) qui sont placés en symétrie miroir dans la direction longitudinale de l'article.

3. Article selon la revendication 1 ou 2, caractérisé en ce que les bords (3) réunis l'un à l'autre des corps (1, 2) sont en forme de S.

4. Article selon l'une quelconque des revendications 1 à 3, caractérisé en ce que les corps (1, 2) sont faits d'un matériau d'enveloppe extérieure, imperméable au liquide, d'un matériau d'enveloppe intérieure perméable aux liquides et destiné à se trouver le plus près du corps de l'utilisateur quand l'article est porté, et d'un coussin absorbant enfermé entre les feuilles d'enveloppe.

5. Article selon l'une quelconque des précédentes revendications, caractérisé en ce que les corps sont deux corps en symétrie miroir (1, 2) ayant un bord commun (3) en forme de S et en ce que les deux parties courbes qui forment le S sont en relation de symétrie miroir par rapport à une ligne passant par les extrémités desdites parties.
